# DEMANDE DE BREVET EUROPEEN

(11) **EP 0 631 217 A1**
(43) Date de publication de la demande: **28.12.1994**
(21) Numéro de dépôt: 94401309.3
(22) Date de dépôt: 10.06.1994
(51) Int. Cl.: G05D 16/20, F04B 49/00, F04D 27/00, G01N 1/14

(54) **Dispositif de régulation de vide**

(30) Priorité: 11.06.1993 FR 9307037
(71) Demandeur: NORMALAB (Société anonyme), F-76210 Bolbec (FR)
(72) Inventeur: Varnier, Samuel Maurice, F-76620 Le Havre (FR)
(74) Mandataire: Derambure, Christian

(57) **Abrégé**

L'invention concerne un dispositif de régulation de vide pour appareils de mesure de la tenue à basse température d'un combustible contenant des paraffines, nécessitant une dépression constante ou variable en fonction du temps, le dispositif comportant un module électronique de commande et une pompe. Selon l'invention, il comprend un moyen de sélection d'au moins un moyen de consigne d'aspiration de la pompe ; un moyen d'atténuation du moyen de consigne d'aspiration notamment relié au module de filtration ; un moyen de mesure de la dépression générée par la pompe ; un moyen de comparaison entre la consigne et la mesure et de modification de la tension appliquée à la pompe si nécessaire.

## Description

L'invention concerne un dispositif de régulation de vide. Plus particulièrement, l'invention concerne un dispositif de régulation de vide destiné à venir s'adapter sur des appareils de mesure de la tenue à basse température d'un combustible contenant des paraffines.

La méthode d'analyse de la tenue à basse température des moteurs à gazoles développée au cours des années soixante, s'est concrétisée par une norme dite Température Limite de Filtrabilité (ou encore Cold Filter Plugging Point, par la suite TLF) régissant de façon draconienne la méthode appliquée ainsi que l'appareillage à utiliser pour mesurer la température limite de filtrabilité des gazoles. L'objet de cette norme était de décrire une méthode pour la détermination de la température la plus élevée à laquelle un volume donné de gazole ne réussit pas à traverser un dispositif de filtration standardisé en un temps déterminé quand il est refroidi sous des conditions standardisées.

Une telle méthode nécessite un appareil comprenant outre un récipient destiné à contenir le combustible à mesurer, un module de refroidissement du combustible, un module de filtration, un dispositif de régulation du vide constitué d'une première bonbonne de 5 litres, partiellement remplie d'eau et d'une deuxième bonbonne de 5 litres vide destinée à servir de réservoir de vide.

Cependant, la tendance de ces dernières années a été d'améliorer les propriétés au froid des gazoles par modification de leur formulation, et notamment par adjonction d'additifs de fluidité. Ainsi, on trouve maintenant des gazoles offrant une opérabilité de plus de 10° C en dessous du point de trouble.

La conséquence de cette tendance est que la norme TLF actuelle ne donne plus de résultats réellement fiables quant à l'opérabilité des gazoles testés.

Une nouvelle méthode d'essai laboratoire dite "Simulated Filter Plugging Point" (SFPP par la suite), a donc été mise au point et fait l'objet du document EP-A-403 097 qui décrit un procédé et un dispositif pour évaluer la performance à basse température d'un combustible contenant des paraffines. Le dispositif comprend notamment un élément filtrant et des moyens permettant d'engendrer un courant de convexion prédéterminé dans le combustible lorsqu'il est contenu dans un récipient, l'élément filtrant étant disposé de façon telle qu'en service, au moins lorsque les moyens de transfert de combustible sont en action, l'élément reçoit préférentiellement des paraffines sédimentées ou en train de se séparer du combustible par sédimentation.

L'élément de filtration est réalisé de telle façon qu'il puisse s'adapter aux appareils TLF mais nécessite contrairement à la méthode TLF une dépression variable en fonction du temps.

C'est pourquoi, en vue d'intéresser le maximum de raffineur à cette méthode, il a été proposé un kit adaptable aux appareils automatiques de TLF actuellement en vigueur. Ce kit est essentiellement constitué d'un module de réglage de la température, d'un module analytique, d'une pompe à vide et de deux flacons à vide d'une capacité de 10 litres.

Il s'ensuit :
- un encombrement physique important des appareils TLF et SFPP du fait de l'utilisation de réservoirs d'air encombrants, de bonbonnes d'eau et de tuyauteries difficiles à implanter occupant beaucoup de surface.
- une mise en place et un transport délicat des appareils.
- la nécessité d'être spécialiste ou tout au moins compétent pour utiliser de tels appareils et notamment pour leur réglage et leur maintenance. S'agissant de la méthode TLF, la dépression dépend directement de la hauteur d'eau. Il est donc nécessaire de surveiller ce niveau et de faire des appoints d'eau régulièrement afin de maintenir la dépression exigée par la norme. Il y lieu aussi de veiller périodiquement au renouvellement de l'eau. S'agissant de l'appareil SFPP, il est nécessaire de veiller à ce que les paramètres de la courbe de dépression soient justes et éviter que la répetabilité de la courbe de dépression se dégrade.

C'est pourquoi, la demande pour des dispositifs de régulation de vide pour appareils de mesure de la tenue à basse température d'un combustible contenant des paraffines, d'utilisation simple et pour lesquels aucune compétence particulière n'est requise pour son utilisation de routine, est actuellement importante.

Un premier objet de l'invention est donc de proposer un dispositif de régulation de vide répondant aux caractéristiques mentionnées ci-dessus.

Plus particulièrement, le but de la présente invention est de proposer un dispositif de régulation de vide dédié à des appareils de mesure de la tenue à basse température d'un combustible contenant des paraffines.

A cet effet, l'invention propose un dispositif de régulation de vide pour appareils de mesure de la tenue à basse température d'un combustible contenant des paraffines nécessitant une dépression constante ou variable en fonction du temps ; ledit dispositif comportant un module électronique de commande et une pompe. Selon l'invention, le dispositif de régulation de vide comprend un moyen de sélection d'au moins un moyen de consigne d'aspiration de la pompe ; un moyen d'atténuation du moyen de consigne d'aspiration, notamment relié au module de filtration ; un moyen de mesure de la dépression générée par la pompe ; un moyen de comparaison entre la consigne et la mesure et de modification de la tension appliquée à la pompe si nécessaire.

Le dispositif de régulation de vide pour appareils de mesure de la tenue à basse température d'un combustible contenant des paraffines, conforme à l'invention, comprend également les caractéristiques suivantes, seules, optionnellement ou en combinaison : il comprend en outre au moins un moyen générateur électronique de consigne variable positionné entre au moins un moyen de consigne d'aspiration constante de la pompe et le moyen de comparaison et de modification ; le moyen générateur de consigne variable comprend des moyens d'établir une courbe de dépression exponentielle ou autre ; la courbe de dépression exponentielle a pour asymptote le moyen de consigne d'aspiration ; le moyen générateur de consigne variable comprend un circuit RC ou un autre moyen électronique analogique ou numérique ; le moyen d'atténuation est constitué par un réservoir dont le volume est compris notamment entre 0,3 litre et 1,5 litre, plus particulièrement de 1 litre ; la pompe est une pompe à clapets, à membranes ou équivalent ; le moyen d'atténuation est constitué par un raccord cylindrique rigide entre la pompe et le module de filtration ; la pompe est une pompe centrifuge ou équivalent ; il comprend un premier moyen de consigne d'aspiration à notamment de l'ordre de 2 kPa et un deuxième moyen de consigne d'aspiration à de l'ordre de 10 à 80 kPa, notamment de l'ordre de 13 kPa ; le deuxième moyen de consigne d'aspiration est la valeur asymptotique du moyen générateur de consigne variable ; il comprend en outre un moyen de décompression permettant la mise à l'atmosphère du moyen d'atténuation ; le moyen générateur de consigne comprend des moyens d'établir une courbe de dépression exponentielle passant par 9 kPa à 10 à 11 secondes après le début d'un test et 13 à 18 kPa à 24 à 28 secondes.

Ainsi, un dispositif de régulation de vide pour appareils de mesure de la tenue à basse température d'un combustible contenant des paraffines du type de l'invention permet :
- d'avoir un système intégré, peu encombrant, autonome.
- d'avoir un dispositif de régulation du vide facilement et rapidement adaptable aux conditions de vide régies par les méthodes TLF et SFPP.
- de s'affranchir des branchements de tuyauterie nécessaires pour passer de la méthode TLF à la méthode SFPP et réciproquement grâce aux moyens de sélection du moyen de consigne.

Il en résulte que le dispositif de régulation de vide de l'invention est à tout instant disponible et simple d'utilisation ; son installation ne nécessite pas de réglage et sa maintenance ne nécessite pas d'opérations compliquées.

Les autres caractéristiques de l'invention seront bien comprises grâce à la description qui suit.

L'invention concerne donc un dispositif de régulation de vide dédié à des appareils de mesure de la tenue à basse température d'un combustible contenant des paraffines.

Ce type d'appareil est destiné à mesurer la température limite de filtrabilité des gazoles selon les méthodes TLF ou SFPP. Il comporte de façon connue notamment au moins un récipient destiné à contenir le combustible à mesurer, au moins un module de refroidissement du combustible et au moins un module de filtration nécessitant une dépression constante ou variable en fonction du temps.

Le module de filtration dépend en effet de la méthode choisie : TLF ou SFPP, la dépression également : constant pour la méthode TLF, variable en fonction du temps pour la méthode SFPP, ladite méthode SFPP s'adaptant sur les appareils TLF actuellement en vigueur.

On comprend donc qu'une caractéristique importante du dispositif de l'invention est la possibilité, selon la méthode choisie, de générer un vide correspondant aux paramètres spécifiés par la méthode choisie.

Cette caractéristique est permise par le fait que le dispositif de l'invention comporte un moyen de sélection d'au moins un moyen de consigne d'aspiration de la pompe ; un moyen d'atténuation du moyen de consigne d'aspiration, notamment reliée au module de filtration ; un moyen de mesure de la dépression générée par la pompe ; un moyen de comparaison entre la consigne et la mesure et de modification de la tension appliquée à la pompe si nécessaire.

Le moyen de sélection permet d'enclencher le moyen de consigne choisi en fonction de la méthode utilisée.

Le ou les moyen(s) de consigne d'aspiration fixe(nt) la valeur de la dépression à atteindre.

Dans une variante préférée de l'invention, le dispositif comprend un premier moyen de consigne d'aspiration à notamment de l'ordre de 2 kPa et un deuxième moyen de consigne d'aspiration constante à de l'ordre de 10 à 80 kPa, notamment de l'ordre de 13 à 18 kPa.

Dans ce cas, le premier moyen de consigne fixe la valeur de la dépression à atteindre pour la mesure de la température limite de filtrabilité des gazoles selon la méthode TLF, valeur qui est constante dans le temps.

Le deuxième moyen de consigne fixe la valeur finale de la dépression à atteindre pour la mesure de la température limite de filtrabilité des gazoles selon la méthode SFPP, méthode qui nécessite une dépression variable en fonction du temps avant d'arriver à ladite valeur finale.

Pour ce faire, le dispositif de régulation du vide selon l'invention comprend en outre au moins un moyen générateur électronique de consigne variable positionné entre au moins un moyen de consigne d'aspiration de la pompe et le moyen de comparaison et de modification.

Dans la variante préférée de l'invention mentionnée ci-dessus, le moyen générateur électronique de consigne variable est alors positionné entre le deuxième moyen de consigne et le moyen de comparaison et de modification.

Le moyen générateur électronique de consigne variable comprend des moyens d'établir une courbe de dépression exponentielle ou autre ayant pour asymptote le moyen de consigne d'aspiration. Dans la variante préférée de l'invention mentionnée ci-dessus, le deuxième moyen de consigne d'aspiration est la valeur asymptotique du moyen générateur de consigne variable. Le dernier comprend des moyens d'établir une courbe de dépression exponentielle passant par 9 kPa à 10 à 11 secondes après le début d'un test et 13 à 18 kPa à 24 à 28 secondes.

Dans une variante préférée de l'invention, le moyen générateur de consigne variable comprend un circuit RC. Mais il peut comprendre en lieu et plan du circuit RC un microprocesseur, des automates, des compteurs ou équivalents et de manière générale, tout autre moyen électronique analogique ou numérique.

Selon l'invention, le dispositif comprend donc également un moyen d'atténuation du moyen de consigne d'aspiration. Ce moyen d'atténuation est notamment relié au module de filtration.

Dans une première variante de l'invention, le moyen d'atténuation est constitué par un réservoir dont le volume est notamment compris entre 0,3 litre et 1,5 litre, plus particulièrement de 1 litre.

Dans ce cas, la pompe est une pompe à clapets à membranes ou équivalents, le réservoir jouant le rôle d'une capacité tampon atténuant les effets de la membrane de la pompe sur la dépression.

Dans une seconde variante de l'invention, le moyen d'atténuation est constituée par un raccord cylindrique rigide entre la pompe et le module de filtration. Dans ce cas, la pompe est une pompe centrifuge.

Le moyen de comparaison et de modification reçoit à tout instant, du moyen de mesure de la dépression générée par la pompe, une mesure qu'il compare immédiatement au moyen de consigne.

Cette comparaison est faite soit entre la mesure et le premier moyen de consigne d'aspiration dans le cas de la méthode TLF, soit entre la mesure et un des points de la courbe de dépression exponentielle générée par le moyen générateur de consigne variable à partir du deuxième moyen de consigne dans le cas de la méthode SFPP.

Le moyen de dépression générée par la pompe ets un moyen permettant de transformer une grandeur physique en grandeur électrique. Ce peut être, à titre non limitatif, un capteur de pression.

Enfin, le dispositif selon l'invention comprend en outre un moyen de décompression permettant la mise à l'atmosphère du moyen d'atténuation.

On comprend donc de tout ce qui précède que l'utilisateur qui désire mesurer la tenue à basse température d'un gazole par à un appareil TLF sur lequel peut venir s'adapter la méthode SFPP pourra immédiatement, en fonction de la méthode choisie (TLF ou SFPP) et donc des conditions de vide imposées par celle-ci, tester le gazole en s'affranchissant d'étapes de préparation longues et fastidieuses, de débranchement de tuyauteries, de réglage des conditions opératoires, tout ceci grâce au dispositif conforme à l'invention.

Ainsi, si l'utilisateur désire tester le combustible par la méthode TLF, il sélectionne grâce au moyen de sélection le premier moyen de consigne d'aspiration correspondant à la méthode TLF. Ce moyen de consigne est alors envoyé via le moyen de comparaison et de modification à la pompe. La pompe agit sur la grandeur physique (vide). A tout instant, le moyen de mesure de la dépression générée par la pompe, positionné au niveau du moyen d'atténuation du moyen de consigne d'aspiration, prend une mesure qui est envoyée au moyen de comparaison et de modification qui compare la mesure prise à la consigne et modifie en conséquence la tension appliquée à la pompe.

Si l'utilisateur désire tester le combustible par la méthode SFPP, il sélectionne grâce au moyen de sélection le deuxième moyen de consigne d'aspiration correspondant à la méthode SFPP. Ce moyen de consigne passe alors par le moyen générateur de consigne variable qui établit la courbe de dépression exponentielle ayant comme asymptote le moyen de consigne. Cette consigne variable est alors envoyée via le moyen de comparaison et de modification à la pompe qui agit en conséquence sur la grandeur physique (vide). A tout instant le moyen de mesure de la dépression générée par la pompe, positionné au niveau du moyen d'atténuation du moyen de consigne d'aspiration, prend une mesure qui est envoyée au moyen de comparaison et de modification qui compare la mesure prise à la consigne et modifie en conséquence la tension appliquée à la pompe.

## Revendications

1. Dispositif de régulation de vide pour appareils de mesure de la tenue à basse température d'un combustible contenant des paraffines, nécessitant une dépression constante ou variable en fonction du temps, ledit dispositif comportant un module électronique de commande et une pompe et étant caractérisé en ce qu'il comprend un moyen de sélection d'au moins un moyen de consigne d'aspiration de la pompe ; un moyen d'atténuation du moyen de consigne d'aspiration notamment relié au module de filtration ; un moyen de mesure de la dépression générée par la pompe ; un moyen de comparaison entre la consigne et la mesure et de modification de la tension appliquée à la pompe si nécessaire ; au moins un moyen générateur électronique de consigne variable positionné entre au moins un moyen de consigne d'aspiration de la pompe et le moyen de comparaison et de modification, le moyen générateur de consigne variable comprenant des moyens d'établir une courbe de dépression exponentielle ou autre.

2. Dispositif selon la revendication 1, caractérisé en ce que la courbe de dépression exponentielle ou autre a pour asymptote le moyen de consigne d'aspiration constante.

3. Dispositif selon la revendication 1 ou 2, caractérisé en ce que le moyen générateur de consigne variable comprend un circuit RC ou un autre moyen électronique analogique ou numérique.

4. Dispositif selon l'une quelconque des revendications 1 à 3, caractérisé en ce que le moyen d'atténaution est constitué par un réservoir dont le volume est compris notamment entre 03 litre et 1,5 litre, plus particulièrement de 1 litre.

5. Dispositif selon la revendication 4, caractérisé en ce que la pompe est une pompe à clapets, à membranes ou équivalent.

6. Dispositif selon l'une quelconque des revendications 1 à 3, caractérisé en ce que le moyen d'atténuation est constitué par un raccord cylindrique rigide entre la pompe et le module de filtration.

7. Dispositif selon la revendication 6, caractérisé en ce que la pompe est une pompe centrifuge ou équivalent.

8. Dispositif selon l'une quelconque des revendications 1 à 7, caractérisé en ce qu'il comprend un premier moyen de consigne d'aspiration à notamment de l'ordre de 2kPa et un deuxième moyen de consigne d'aspiration à de l'ordre de 10 à 80 kPa, notamment de l'ordre de 13 kPa.

9. Dispositif selon la revendication 8, caractérisé en ce que le deuxième moyen de consigne d'aspiration est la valeur asymptotique du moyen générateur de consigne variable.

10. Dispositif selon la revendication 9, caractérisé en ce que le moyen générateur de consigne comprend des moyens d'établir une courbe de dépression exponentielle passant par 9 kPa à 10 à 11 secondes après le début d'un test et 13 à 18 kPa à 24 à 28 secondes.

11. Dispositif selon ll'une quelconque des revendications 1 à 10, caractérisé en ce qu'il comprend en outre un moyen de décompression permettant la mise à l'atmosphère du moyen d'atténaution.
